# EUROPEAN PATENT APPLICATION

(11) **EP 4 582 080 A1**
(43) Date of publication of application: **09.07.2025**
(21) Application number: 24150010.7
(22) Date of filing: 02.01.2024
(51) Int. Cl.: A61K 9/08, A61K 31/7004, A61P 31/16

(54) **DISPENSER FOR MUCOSAL ADMINISTRATION COMPRISING 2-DEOXY-GLUCOSE OR ANALOGUES THEREOF**

(71) Applicant: G.ST Antivirals GmbH, 1030 Wien (AT)
(72) Inventor: GUALDONI, Guido, 1030 Wien (AT); GORKI, Anna-Dorothea, 1030 Wien (AT); RADIVOJEV, Snezana, 1030 Wien (AT); BERNKOP-SCHNÜRCH, Andreas, 6020 Innsbruck (AT)
(74) Representative: SONN Patentanwälte GmbH & Co KG

(57) **Abstract**

Disclosed is a pharmaceutical dispenser for mucosal administration, preferably intranasal or inhalatory administration, containing a pharmaceutical composition. The pharmaceutical composition comprises an active pharmaceutical ingredient (API) selected from the group consisting of 2-deoxy-glucose (2-DG) and analogues thereof, or a pharmaceutically acceptable salt of the API. The pH of the pharmaceutical composition is lower than 7.4, optionally upon reconstitution in water. Also disclosed is a method for mucosally administering this pharmaceutical composition, preferably for preventing or treating an infection.

## Description

The field of the present invention relates to pharmaceutical dispensers for mucosal administration, preferably nasal or inhalatory administration, comprising 2-deoxy-glucose (2-DG) or analogues thereof.

Such dispensers are known for instance from WO 2019/149436 A1. Specifically, the document discloses a pharmaceutical composition for prevention or treatment of the common cold. The composition comprises an aldohexose, wherein the hydroxyl group at carbon 2 of the aldohexose is replaced by any one of H, F, CI, Br, I, SH, Me, OMe and SMe, such as a 2-DG. A dispenser for intranasal administration, such as a nasal spray or nose drop applicator containing said pharmaceutical composition is disclosed. In addition, an inhalation device, such as a metered-dose inhaler, a dry-powder inhaler or a nebuliser, comprising said composition is disclosed.

Such dispensers are also known e.g., from WO 2021/205412 A1 and WO 2023/062516 A1, both of which relate to formulations of 2-DG with liposomes, among others.

2-DG and analogues may be used for the treatment of infections. They have also been suggested as anticancer agents.

There is an ongoing need for formulations of 2-DG and analogues with improved pharmacological properties when administered to a mucosa of a patient (e.g., the nasal mucosa or a mucosa of the respiratory tract), such as increased safety and/or efficacy.

It is thus an object of the present invention to provide pharmaceutical dispensers for mucosal administration comprising such improved formulations of 2-DG or analogues thereof.

The present invention provides a pharmaceutical dispenser for mucosal administration, preferably intranasal or inhalatory administration, containing a pharmaceutical composition. The pharmaceutical composition comprises an active pharmaceutical ingredient (API) selected from the group consisting of 2-deoxy-glucose ("2-DG", which preferably relates to 2-deoxy-D-glucose herein) and analogues thereof (as defined hereinbelow), or a pharmaceutically acceptable salt of the API. Importantly, the pH of the pharmaceutical composition is lower than 7.4, optionally upon reconstitution in (deionized or distilled) water (e.g., when the pharmaceutical composition is formulated as a powder).

In an aspect, the present invention relates to the pharmaceutical composition as defined above for use in prevention or treatment of an infection (in particular a respiratory tract infection), wherein the composition is administered mucosally. Preferably, the pharmaceutical composition is for use in prevention or treatment of the common cold, a rhinitis or a lower respiratory tract infection, especially in an immunosuppressed individual or in an individual having chronic obstructive pulmonary disease (COPD), cystic fibrosis or asthma.

In another aspect, the present invention provides a method for mucosally administering a pharmaceutical composition, the method comprising the steps of obtaining the pharmaceutical composition, and mucosally administering the composition to an individual having an infection or at risk of developing an infection; wherein the pharmaceutical composition comprises an API selected from the group consisting of 2-DG and analogues thereof (as defined hereinbelow), or a pharmaceutically acceptable salt of the API. Importantly, the pH of the pharmaceutical composition is lower than 7.4, optionally upon reconstitution in water (e.g., when the pharmaceutical composition is formulated as a powder).

In the course of the present invention, it was surprisingly found that the 2-DG and analogues interacted with free amines in the mucus, leading to the formation of Schiff's bases (imines). This reduced bioavailability of 2-DG and analogues to the mucosal epithelial cells. This undesired effect appears to be particularly pronounced when the mucus is thick, as is the case e.g., with certain respiratory tract infections. This undesired effect appears already when the mucosa is exposed only briefly to the drug product, as is common with certain forms of mucosal administration such as intranasal administration, in particular with nasal sprays.

Several approaches were attempted to address this undesirable interaction between 2-DG and analogues with the mucus. Surprisingly, it turned out that reducing the pH of the formulation improved mucus penetration, thereby increasing bioavailability. This in turn enhances the efficacy of a formulation for mucosal administration at a given concentration of 2-DG (or analogues thereof).

While acidic pH ranges are clearly preferred for the pharmaceutical composition due to increased bioavailability (cf. Example 1), under certain circumstances, a pH up to just below 7.4 may be tolerable with respect to imine formation (especially for mucosae with thin mucus). However, it is essential that the pH of the pharmaceutical composition is lower than 7.4.

Preferably, the pH of the pharmaceutical composition is lower than 7.2, preferably lower than 7.0 or even lower than 6.8, more preferably lower than 6.8 or even lower than 6.6, even more preferably lower than 6.4 or even lower than 6.2, yet even more preferably lower than 6.0 or even lower than 5.8, especially lower than 5.6 or even lower than 5.4; optionally upon reconstitution in water.

According to a particularly preferred embodiment, the pH of the pharmaceutical composition is 3.0 to 7.0, preferably 3.2 to 6.8 or even 3.4 to 6.6, more preferably 3.6 to 6.4 or even 3.8 to 6.2, even more preferably 4.0 to 6.0.

Typically, to better maintain the pH in the preferred range, the pharmaceutical composition comprises a (pharmaceutically acceptable) buffering agent. In a preferred embodiment, the buffering agent comprises a weak acid with a pKₐ of 3.0 to 7.0, preferably 3.5 to 6.5, more preferably 4.0 to 6.0, especially 4.5 to 5.5.

According to a particularly preferred embodiment, the buffering agent is selected from the group consisting of citrate, phosphate, acetate, citrate/phosphate, tris(hydroxymethyl)aminomethane (Tris), Tris/glycine, Tris/acetate, maleate, tartrate, boric acid/borate, ascorbate, succinate and phthalate. Particularly preferred buffering agents are citrate, citrate/phosphate, tartrate and maleate.

In another preference, the concentration of the buffering agent in the pharmaceutical composition is 5 mM to 250 mM, preferably 7.5 mM to 100 mM, more preferably 10 mM to 50 mM, especially 12.5 mM to 25 mM.

As used herein, all 2-DG analogues have structural formula I 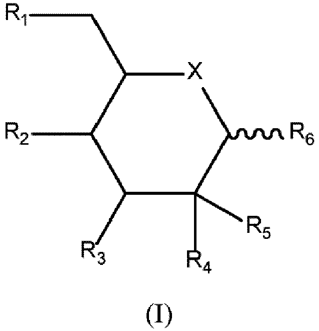 wherein:
X is selected from the group consisting of O and S,
R₁, R₂, R₃, and R₆ are independently selected from the group consisting of hydrogen, hydroxyl, thiol, halogen, alkoxy, haloalkoxy, perhaloalkoxy, alkoxyalkyloxy, -OC(O)alkyl, OCO₂alkyl, alkylthio, amino, alkylamino, N-sulfonamido, N-amido, and carbamate, any of which may be optionally substituted,
R₄ and R₅ are independently selected from the group consisting of hydrogen, hydroxyl, thiol, halogen, alkoxy, haloalkoxy, per haloalkoxy, alkoxyalkyloxy, -OC(O)alkyl, OCO₂alkyl, alkylthio, amino, alkylamino, N-sulfonamido, N-amido, azido, carbamate, alkyl, haloalkyl, perhaloalkyl, -N(R₇)OR₈, - ON(R₉)₂, -N(R₁₀)N(R₁₁)₂, any of which may be optionally substituted, or R₄ and R₅, taken together, are selected from the group consisting of =N-OR₁₂ and =N-N(R₁₃)₂, and
R₇, R₈, R₉, R₁₀, R₁₁, R₁₂, and R₁₃ are independently selected from the group consisting of hydrogen and alkyl, wherein said alkyl may be optionally substituted,
with the proviso that if X is O and R₄ and R₅ are both H, then at least one of R₁, R₂, R₃, and R₆ is not OH.
2-DG analogues may for instance be synthesized according to the procedures described in US 8,927,506 B2 (columns 14-26); WO 2010005799 (paragraphs [0086]-[0145]); WO 2009108926 (paragraphs [0173]-[0185]); WO 2008131024 (paragraphs [0067]-[0072]); US 20100152121 (paragraphs [0067]-[0083]); US 7,160,865 (columns 11-13); and US 6,979,675 (columns 28-29), the disclosures of which are incorporated herein by reference.

According to a preference, the 2-DG analogues have structural formula II 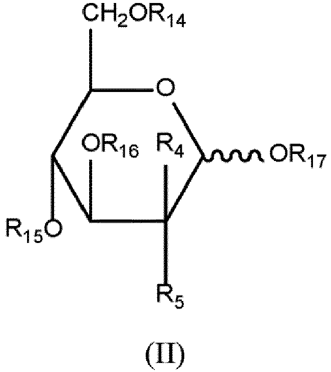 wherein:
R₁₄, R₁₅, R₁₆, and R₁₇ are independently selected from the group consisting of hydrogen, COCH₃, COCH₂CH₃, and COCH₂CH₂CH₃; and
R₄ and R₅ are independently selected from the group consisting of H, Cl, Br, I, F, SH, Me, Ome and Sme, with the proviso that if R₄ and R₅ are both H, then at least one of R₁₄, R₁₅, R₁₆, and R₁₇ is not H.

These 2-DG analogues and/or 2-DG are preferred as the API.

Further preferred APIs are the 2-DG analogues with structural formulas III or IV: 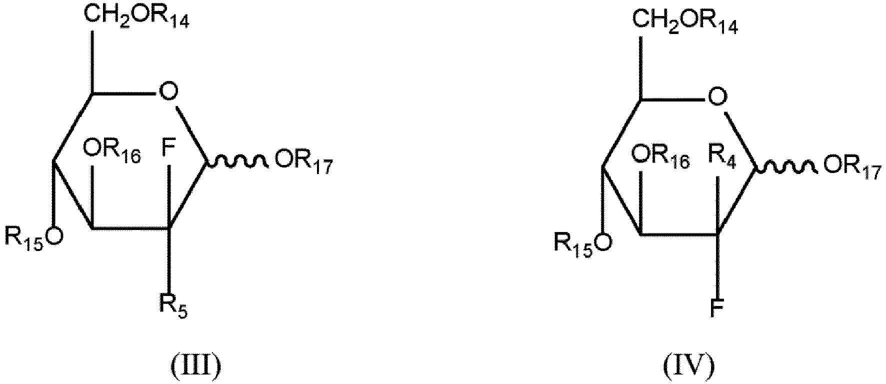 wherein:
R₁₄, R₁₅, R₁₆, and R₁₇ are independently selected from the group consisting of hydrogen, COCH₃, COCH₂CH₃, and COCH₂CH₂CH₃ (in particular wherein R₁₄, R₁₅, R₁₆, and R₁₇ are hydrogen),
R₄ and R₅ are independently selected from the group consisting of hydrogen, hydroxyl, thiol, halogen (in particular F), alkoxy, haloalkoxy, per haloalkoxy, alkoxyalkyloxy, - OC(O)alkyl, OCO₂alkyl, alkylthio, amino, alkylamino, N-sulfonamido, N-amido, azido, carbamate, alkyl, haloalkyl, perhaloalkyl, -N(R₇)OR₈, -ON(R₉)₂, -N(R₁₀)N(R₁₁)₂, any of which may be optionally substituted, or R₄ and R₅, taken together, are selected from the group consisting of =N-OR₁₂ and =N-N(R₁₃)₂, and
R₇, R₈, R₉, R₁₀, R₁₁, R₁₂, and R₁₃ are independently selected from the group consisting of hydrogen and alkyl, wherein said alkyl may be optionally substituted.

In another preferred embodiment, the API is any one of compounds V-XII (depicted as Fischer projections): 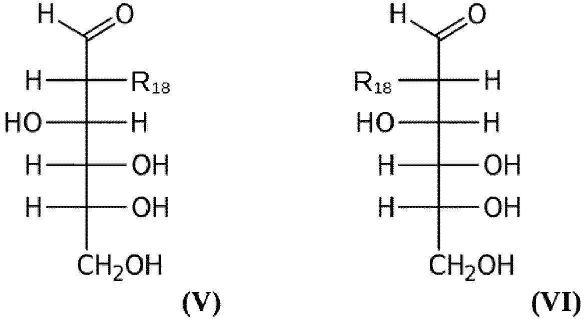 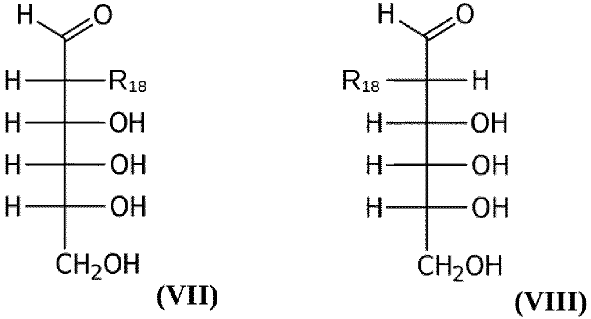 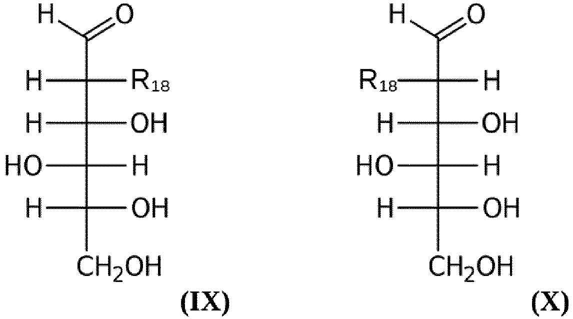 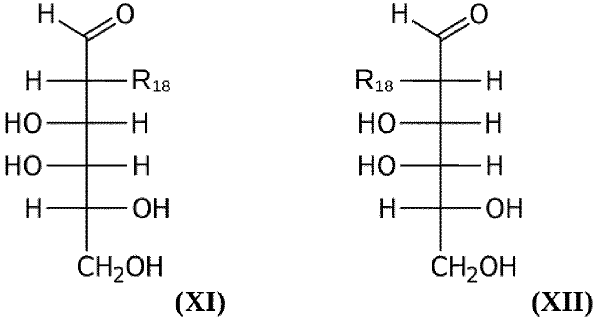 wherein R₁₈ is any one of H, F, Cl, Br, I, SH, Me, OMe, azido and SMe, preferably H, F, Cl, Br, I, and F, in particular H and F.

Compounds V (i.e., the compound based on D-glucose) and VI (i.e., the compound based on D-mannose) are particularly preferred 2-DG analogues, preferably when R₁₈ is F, Cl, Br, I, especially F.

According to another preferred embodiment, the API is an aldohexose, preferably a D-aldohexose, wherein the hydroxyl group at carbon 2 of the aldohexose is replaced by any one of H, F, Cl, Br, I, SH, Me, OM, SMe and azido. It is especially preferred when the aldohexose is a mannose or glucose, preferably D-glucose or D-mannose.

In a particularly preferred embodiment, the API is selected from the group consisting of 2-DG, 2-chloro-2-deoxy-glucose, 2-fluoro-2-deoxy-glucose, 2-azido-2-deoxy-glucose, 2-chloro-2-deoxy-mannose, 2-fluoro-2-deoxy-mannose and 2-azido-2-deoxy-mannose, in particular selected from the group consisting of 2-DG, 2-fluoro-2-deoxy-glucose and 2-fluoro-2-deoxy-mannose; preferably wherein the glucose is D-glucose and the mannose is D-mannose.

According to another particularly preferred embodiment, the API is selected from the 2-DG analogues given in examples 1-84 of paragraph [0151] of WO 2021/188586 A1. These examples 1-84 are incorporated herein by reference.

In another preferred embodiment, the pharmaceutical dispenser for mucosal administration is a dispenser for intranasal administration, preferably a nasal spray or a nose drop applicator. Nasal sprays and nose drop applicators are known in the art and e.g., disclosed in US patent no. 2,577,321, US patent no. 6,000,580 or EP 0 170 198 A2. As used herein, the term "nose drop applicator" refers to any pharmaceutical dispenser intended for the administration of nose drops.

According to yet another preference, the pharmaceutical dispenser for mucosal administration is an inhalation device. This inhalation device is preferably a metered-dose inhaler, a dry-powder inhaler or a nebuliser.

A metered-dose inhaler is a device that aerosolises a predefined dose of a pharmaceutical composition (i.e., produces a comparatively short burst of aerosol with a defined dose), usually for self-administration by the patient. A metered-dose inhaler is for instance disclosed in US 6,260,549 E1.

Dry-powder inhalers are devices for inhalation of dry-powder formulations by the patient. Such devices are for instance disclosed in US Patents 4,995,385 and 4,069,819. Established dry-powder inhalers are for instance SPINHALER^{®}, ROTAHALER^{®}, FLOWCAPS^{®}, INHALATOR^{®}, DISKHALER^{®} and AEROLIZER^{®}.

Nebulisers are devices that produce aerosols for inhalation, typically continuously as long as they are switched on or breath-actuated. Established nebuliser products are for instance Aeroneb^{®} and Pari^{®}. Document US 9,364,618 B2, e.g., also discloses a nebuliser.

In the context of the present invention, the expression "pharmaceutical composition" refers to any composition comprising the API (i.e., 2-DG or an analogue thereof as disclosed herein) or a pharmaceutically acceptable salt thereof, typically further comprising a buffering agent (preferably a weak acid with a pKₐ of 3.0 to 7.0, preferably 3.5 to 6.5, more preferably 4.0 to 6.0, especially 4.5 to 5.5) and preferably one or more excipients, which composition is pharmaceutically acceptable for mucosal administration (in particular intranasal or inhalatory administration) to an individual, especially a mammal, in particular a human. Suitable excipients are known to the person skilled in the art, for example water (especially water for injection), saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives, such as benzalkonium chloride. The pharmaceutical composition according to the present invention may be liquid or ready to be dissolved in liquid such as sterile, deionised or distilled water or a buffer solution. The dosage and method of administration, typically depends on the individual to be treated. In general, the API (in particular 2-DG) may be administered at a dose of between 1 pg/kg and 10 mg/kg, more preferably between 10 pg/kg and 5 mg/kg, most preferably between 0.1 and 2 mg/kg.

It is preferred that pharmaceutical composition comprises a preservative, preferably selected from the group consisting of benzalkonium chloride, phenylcarbinol, povidone iodine, EDTA, propylene glycol, polyethylene glycol, potassium sorbate, polysorbate 80, Tween 80, chlorobutanol, eucalyptus oil, citrus oil, peppermint oil, phytic acid, chelating agents and zinc. The concentration (w/w) of the preservative in the pharmaceutical composition may be 0.005% to 2.0%, preferably 0.05% to 1.5%, more preferably 0.1% to 1.0%.

According to a particularly preferred embodiment of the present invention, the pharmaceutical composition is liquid and preferably an aqueous solution. Liquid compositions are especially suitable for intranasal or inhalatory administration, which are the preferred modes of administration.

In a further preferment, the concentration of the API, in particular 2-DG, is 10 mM - 2000 mM, preferably 20 mM - 1000 mM, more preferably 50 mM - 900 mM, even more preferably 100 mM - 800 mM, in particular 200 mM - 700 mM. These concentration ranges turned out to be safe and effective in connection with the treatment of infections, in particular intranasal treatment.

The pharmaceutical composition may comprise further active agents, in particular to further increase effectiveness or achieve further symptom reduction of the common cold or other respiratory infections. Thus, according to a further preferred embodiment, the pharmaceutical composition further comprises at least one additional active agent. Preferably, the additional active agent is selected from the group consisting of decongestants, in particular norepinephrine releasing agents (such as pseudoephedrine, ephedrine and phenylpropanolamine), α-adrenergic receptor agonists (such as oxymetazoline and xylometazoline), and corticosteroids (such as budesonide, flunisolide, and fluticasone), and nonsteroidal anti-inflammatory drugs (NSAIDs) such as acetylsalicylic acid, ibuprofen, diclofenac, and phenylbutazone. The pharmaceutical composition may also comprise an additional antiviral agent.

Alternatively, the API such as 2-DG may also be the single active agent in the pharmaceutical composition (preferably in the presence of a buffering agent and one or more excipients).

The individual to be treated according to the present invention is preferably a human individual, in particular an immunosuppressed individual or an individual having chronic COPD, cystic fibrosis or asthma. According to a preferred embodiment, a dose of the pharmaceutical composition is administered to a human individual, preferably intranasally (in other words: into a nostril of the individual, preferably into each nostril independently) or to another mucosal membrane, preferably another mucosal membrane of the respiratory tract, in particular a mucosal membrane of the lower respiratory tract (e.g., by inhalation). Advantageously, the dose is administered at least once every other day, preferably at least once per day, more preferably at least twice per day, in particular at least thrice per day or even at least four times per day, and preferably for 2 - 14 days, more preferably for 3 - 10 days, in particular for 4 - 7 days. In embodiments, the dose may be administered (prophylactically) throughout the cold season (or period of increased respiratory virus activity), e.g. for at least two weeks or even at least 1 month, preferably for at least 2 months, especially for (at least) 3 months.

For relatively safe and effective treatment of respiratory infections (e.g., the common cold), according to a preference, the pharmaceutical dispenser is a nasal spray, wherein the API (in particular 2-DG) dose of one spray into a nostril is 10 pmol - 2000 pmol, preferably 20 pmol - 1500 pmol, more preferably 50 pmol - 1000 pmol, even more preferably 100 pmol - 500 pmol, in particular 150 pmol - 350 pmol.

### Further definitions

The term "preventing" or "prevention" as used herein means to stop a disease state or condition from occurring in an individual completely or almost completely or at least to a (preferably significant) extent, especially when the individual is predisposed to such a risk of contracting a disease state or condition.

The term "alkoxy," as used herein, alone or in combination, refers to an alkyl ether radical, wherein the term alkyl is as defined below. Examples of suitable alkyl ether radicals include methoxy, ethoxy, n-propoxy, isopropoxy, n-butoxy, iso-butoxy, sec-butoxy, tert-butoxy, and the like.

The term "alkyl," as used herein, alone or in combination, refers to a straight-chain or branched-chain alkyl radical containing from 1 to 20 carbon atoms. In certain embodiments, said alkyl will comprise from 1 to 10 carbon atoms. In further embodiments, said alkyl will comprise from 1 to 6 carbon atoms. In further embodiments, said alkyl will comprise 1 to 3 carbon atoms. Alkyl groups may be optionally substituted as defined herein. Examples of alkyl radicals include methyl, ethyl, n-propyl, isopropyl, n-butyl, isobutyl, sec- butyl, tert-butyl, pentyl, iso-amyl, hexyl, octyl, noyl and the like. The term "alkylene," as used herein, alone or in combination, refers to a saturated aliphatic group derived from a straight or branched chain saturated hydrocarbon attached at two or more positions, such as methylene (-CH₂-). Unless otherwise specified, the term "alkyl" may include "alkylene" groups.

The term "alkylamino," as used herein, alone or in combination, refers to an alkyl group attached to the parent molecular moiety through an amino group. Suitable alkylamino groups may be mono- or dialkylated, forming groups such as, for example, N-methylamino, N-ethylamino, N,N-dimethylamino, N,N-ethylmethylamino and the like.

The term "alkylthio," as used herein, alone or in combination, refers to an alkyl thioether (R-S-) radical wherein the term alkyl is as defined above and wherein the sulfur may be singly or doubly oxidized. Examples of suitable alkyl thioether radicals include methylthio, ethylthio, n-propylthio, isopropylthio, n-butylthio, iso-butylthio, sec-butylthio, tert-butylthio, methanesulfonyl, ethanesulfmyl, and the like.

The term "amino," as used herein, alone or in combination, refers to -NRR' wherein R and R are independently selected from the group consisting of hydrogen, alkyl, acyl, heteroalkyl, aryl, cycloalkyl, heteroaryl, and heterocycloalkyl, any of which may themselves be optionally substituted. Additionally, R and R' may combine to form heterocycloalkyl, either of which may be optionally substituted.

The term "carbamate," as used herein, alone or in combination, refers to an ester of carbamic acid (-NHCOO-) which may be attached to the parent molecular moiety from either the nitrogen or acid end, and which may be optionally substituted as defined herein.

The term "cycloalkyl," or, alternatively, "carbocycle," as used herein, alone or in combination, refers to a saturated or partially saturated monocyclic, bicyclic or tricyclic alkyl group wherein each cyclic moiety contains from 3 to 12 carbon atom ring members and which may optionally be a benzo fused ring system which is optionally substituted as defined herein. In certain embodiments, said cycloalkyl will comprise from 5 to 7 carbon atoms. Examples of such cycloalkyl groups include cyclopropyl, cyclobutyl, cyclopentyl, cyclohexyl, cycloheptyl, tetrahydronapthyl, indanyl, octahydronaphthyl, 2, 3 -dihydro-1H- indenyl, adamantyl and the like. "Bicyclic" and "tricyclic" as used herein are intended to include both fused ring systems, such as decahydronaphthalene, octahydronaphthalene as well as the multicyclic (multi centered) saturated or partially unsaturated type. The latter type of isomer is exemplified in general by, bicyclo[l,l,l]pentane, camphor, adamantane, and bicyclo[3,2,1]octane.

The term "haloalkoxy," as used herein, alone or in combination, refers to a haloalkyl group attached to the parent molecular moiety through an oxygen atom.

The term "haloalkyl," as used herein, alone or in combination, refers to an alkyl radical having the meaning as defined above wherein one or more hydrogens are replaced with a halogen. Specifically embraced are monohaloalkyl, dihaloalkyl and polyhaloalkyl radicals. A monohaloalkyl radical, for one example, may have an iodo, bromo, chloro or fluoro atom within the radical. Dihalo and polyhaloalkyl radicals may have two or more of the same halo atoms or a combination of different halo radicals. Examples of haloalkyl radicals include fluoromethyl, difluoromethyl, trifluoromethyl, chloromethyl, dichloromethyl, trichloromethyl, pentafluoroethyl, heptafluoropropyl, difluorochloromethyl, dichlorofluoromethyl, difluoroethyl, difluoropropyl, dichloroethyl and dichloropropyl.

The term "perhaloalkoxy," refers to an alkoxy group where all of the hydrogen atoms are replaced by halogen atoms.

The term "perhaloalkyl," as used herein, alone or in combination, refers to an alkyl group where all of the hydrogen atoms are replaced by halogen atoms.

The term "optionally substituted" means the anteceding group may be substituted or unsubstituted. When substituted, the substituents of an "optionally substituted" group may include, without limitation, one or more substituents independently selected from the following groups or a particular designated set of groups, alone or in combination: lower alkyl, lower alkenyl, lower alkynyl, lower alkanoyl, lower heteroalkyl, lower heterocycloalkyl, lower haloalkyl, lower haloalkenyl, lower haloalkynyl, lower perhaloalkyl, lower perhaloalkoxy, lower cycloalkyl, phenyl, aryl, aryloxy, lower alkoxy, lower haloalkoxy, oxo, lower acyloxy, carbonyl, carboxyl, lower alkylcarbonyl, lower carboxyester, lower carboxamido, cyano, hydrogen, halogen, hydroxy, amino, lower alkylamino, arylamino, amido, nitro, thiol, lower alkylthio, lower haloalkylthio, lower perhaloalkylthio, arylthio, sulfonate, sulfonic acid, tri substituted silyl, N₃, SH, SCH₃, C(O)CH₃, CO₂CH₃, CO₂H, pyridinyl, thiophene, furanyl, lower carbamate, and lower urea. In this context, the term "lower" means containing from 1 to and including 6 carbon atoms. Preferably, the term "lower" when describing an alkyl moiety refers to 1- 3 carbon atoms. Two substituents may be joined together to form a fused five-, six-, or seven-membered carbocyclic or heterocyclic ring consisting of zero to three heteroatoms, for example forming methylenedioxy or ethylenedioxy. An optionally substituted group may be unsubstituted (e.g., -CH₂CH₃), fully substituted (e.g., -CF₂CF₃), monosub stituted (e.g., -CH₂CH₂F) or substituted at a level anywhere in-between fully substituted and monosub stituted (e.g., -CH₂CF₃). Where substituents are recited without qualification as to substitution, both substituted and unsubstituted forms are encompassed. Where a substituent is qualified as "substituted" the substituted form is specifically intended. Additionally, different sets of optional substituents to a particular moiety may be defined as needed; in these cases, the optional substitution will be as defined, often immediately following the phrase, "optionally substituted with."

Asymmetric centers may exist in the APIs disclosed herein. It should be understood that the invention encompasses all stereochemical isomeric forms, including diastereomeric, enantiomeric, and epimeric forms, as well as d-isomers and 1-isomers, and mixtures thereof. Individual stereoisomers of compounds can be prepared synthetically from commercially available starting materials which contain chiral centers or by preparation of mixtures of enantiomeric products followed by separation such as conversion to a mixture of diastereomers followed by separation or recrystallization, chromatographic techniques, direct separation of enantiomers on chiral chromatographic columns, or any other appropriate method known in the art. Starting compounds of particular stereochemistry are either commercially available or can be made and resolved by techniques known in the art.

Certain APIs disclosed herein may also exist as prodrugs, as for instance described in Hydrolysis in Drug and Prodrug Metabolism: Chemistry, Biochemistry, and Enzymology (Testa, Bernard and Mayer, Joachim M. Wiley-VHCA, Zurich, Switzerland 2003). Prodrugs of the APIs described herein are structurally modified forms of the compound that readily undergo chemical changes under physiological conditions to provide the API. Additionally, prodrugs can be converted to the API by chemical or biochemical methods in an ex vivo environment.

The present invention further relates to the following embodiments:
Embodiment 1. A pharmaceutical dispenser for mucosal administration, preferably intranasal or inhalatory administration, containing a pharmaceutical composition,
   wherein the pharmaceutical composition comprises an active pharmaceutical ingredient (API) selected from the group consisting of 2-deoxy-glucose (2-DG) and analogues thereof, or a pharmaceutically acceptable salt of the API,
   wherein the pH of the pharmaceutical composition is lower than 7.4, optionally upon reconstitution in water;
   wherein the analogues have structural formula I 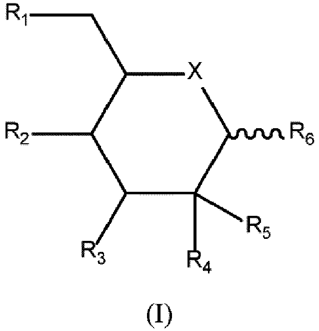
   wherein:
      X is selected from the group consisting of O and S,
      R₁, R₂, R₃, and R₆ are independently selected from the group consisting of hydrogen, hydroxyl, thiol, halogen, alkoxy, haloalkoxy, perhaloalkoxy, alkoxyalkyloxy, -OC(O)alkyl, OCO₂alkyl, alkylthio, amino, alkylamino, N-sulfonamido, N-amido, and carbamate, any of which may be optionally substituted,
      R₄ and R₅ are independently selected from the group consisting of hydrogen, hydroxyl, thiol, halogen, alkoxy, haloalkoxy, per haloalkoxy, alkoxyalkyloxy, -OC(O)alkyl, OCO₂alkyl, alkylthio, amino, alkylamino, N-sulfonamido, N-amido, azido, carbamate, alkyl, haloalkyl, perhaloalkyl, -N(R₇)OR₈, -ON(R₉)₂, - N(R₁₀)N(R₁₁)_{2'} any of which may be optionally substituted, or R₄ and R₅, taken together, are selected from the group consisting of =N-OR₁₂ and =N-N(R₁₃)₂, and
      R₇, R₈, R₉, R₁₀, R₁₁, R₁₂, and R₁₃ are independently selected from the group consisting of hydrogen and alkyl, wherein said alkyl may be optionally substituted,
      with the proviso that if X is O and R₄ and R₅ are both H, then at least one of R₁, R₂, R₃, and R₆ is not OH.
Embodiment 2. The pharmaceutical dispenser of embodiment 1, wherein the pharmaceutical dispenser is selected from the group consisting of dispensers for intranasal administration, such as nasal sprays and nose drop applicators, and inhalation devices, such as dry-powder inhalers, metered-dose inhalers and nebulizers.
Embodiment 3. The pharmaceutical dispenser of embodiment 2, wherein the pharmaceutical dispenser is a nasal spray and the pharmaceutical composition is an aqueous solution.
Embodiment 4. The pharmaceutical dispenser of any one of embodiments 1 to 3, wherein the pH of the pharmaceutical composition is lower than 7.2, preferably lower than 7.0 or even lower than 6.8, more preferably lower than 6.8 or even lower than 6.6, even more preferably lower than 6.4 or even lower than 6.2, yet even more preferably lower than 6.0 or even lower than 5.8, especially lower than 5.6 or even lower than 5.4; optionally upon reconstitution in water.
Embodiment 5. The pharmaceutical dispenser of embodiment 4, wherein the pH of the pharmaceutical composition is 3.0 to 7.0, preferably 3.2 to 6.8 or even 3.4 to 6.6, more preferably 3.6 to 6.4 or even 3.8 to 6.2, even more preferably 4.0 to 6.0.
Embodiment 6. The pharmaceutical dispenser of any one of embodiments 1 to 5, wherein the pharmaceutical composition comprises a buffering agent.
Embodiment 7. The pharmaceutical dispenser of embodiment 6, wherein the buffering agent comprises a weak acid with a pKₐ of 3.0 to 7.0, preferably 3.5 to 6.5, more preferably 4.0 to 6.0, especially 4.5 to 5.5.
Embodiment 8. The pharmaceutical dispenser of embodiment 6 or 7, wherein the buffering agent is selected from the group consisting of citrate, phosphate, acetate, citrate/phosphate, tris(hydroxymethyl)aminomethane (Tris), Tris/glycine, Tris/acetate, maleate, tartrate, boric acid/borate, ascorbate, succinate and phthalate, in particular citrate, citrate/phosphate and tartrate.
Embodiment 9. The pharmaceutical dispenser of any one of embodiments 6 to 8, wherein the pharmaceutical composition is an aqueous solution, wherein the concentration of the buffering agent in the pharmaceutical composition is 5 mM to 250 mM, preferably 7.5 mM to 100 mM, more preferably 10 mM to 50 mM, especially 12.5 mM to 25 mM.
Embodiment 10. The pharmaceutical dispenser of any one of embodiments 1 to 9, wherein the pharmaceutical composition comprises a preservative, preferably selected from the group consisting of benzalkonium chloride, phenylcarbinol, povidone iodine, EDTA, propylene glycol, polyethylene glycol, potassium sorbate, polysorbate 80, Tween 80, chlorobutanol, eucalyptus oil, citrus oil, peppermint oil, phytic acid, chelating agents and zinc.
Embodiment 11. The pharmaceutical dispenser of any one of embodiments 1 to 10, wherein the concentration (w/w) of the preservative in the pharmaceutical composition is 0.005% to 2.0%, preferably 0.05% to 1.5%, more preferably 0.1% to 1.0%.
Embodiment 12. The pharmaceutical dispenser of any one of embodiments 1 to 11, wherein the pharmaceutical composition is an aqueous solution, wherein the concentration of the API in the pharmaceutical composition is 10 mM - 2000 mM, preferably 20 mM - 1000 mM, more preferably 50 mM - 900 mM, even more preferably 100 mM - 800 mM, in particular 200 mM - 700 mM.
Embodiment 13. The pharmaceutical dispenser of any one of embodiments 1 to 12, wherein the pharmaceutical dispenser is a nasal spray, wherein the API dose of one spray into a nostril is 10 pmol - 2000 pmol, preferably 20 pmol - 1500 pmol, more preferably 50 pmol - 1000 pmol, even more preferably 100 pmol - 500 pmol, in particular 150 pmol - 350 pmol.
Embodiment 14. The pharmaceutical dispenser of any one of embodiments 1 to 13, wherein the pharmaceutical composition further comprises an additional active agent, preferably wherein the additional active agent is selected from the group consisting of decongestants, in particular norepinephrine releasing agents, α-adrenergic receptor agonists, and corticosteroids, and nonsteroidal anti-inflammatory drugs, especially wherein pharmaceutical dispenser is a dispenser for intranasal administration such as a nasal spray or a nose drop applicator, or wherein the additional active agent is an antiviral agent.
Embodiment 15. The pharmaceutical dispenser of any one of embodiments 1 to 14, wherein the pharmaceutical composition is liquid, preferably wherein the pharmaceutical composition is an aqueous solution.
Embodiment 16. The pharmaceutical dispenser of any one of embodiments 1 to 15, wherein the analogues have structural formula II 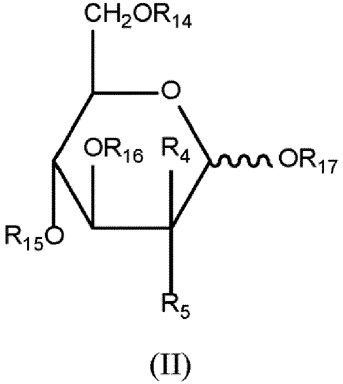 wherein:
   R₁₄, R₁₅, R₁₆, and R₁₇ are independently selected from the group consisting of hydrogen, COCH₃, COCH₂CH₃, and COCH₂CH₂CH₃; and
   R₄ and R₅ are independently selected from the group consisting of H, Cl, Br, I, F, SH, Me, OMe and SMe, with the proviso that if R₄ and R₅ are both H, then at least one of R₁₄, R₁₅, R₁₆, and R₁₇ is not H.
Embodiment 17. The pharmaceutical dispenser of embodiment 16, wherein R₄ and R₅ are independently selected from the group consisting of H, Cl, Br, I and F, preferably H, Cl and F.
Embodiment 18. The pharmaceutical dispenser of embodiment 16 or 17, wherein R₁₄, R₁₅, R₁₆, and R₁₇ are independently selected from the group consisting of hydrogen and COCH₃.
Embodiment 19. The pharmaceutical dispenser of any one of embodiments 1 to 15, wherein the analogues have structural formula III or IV: 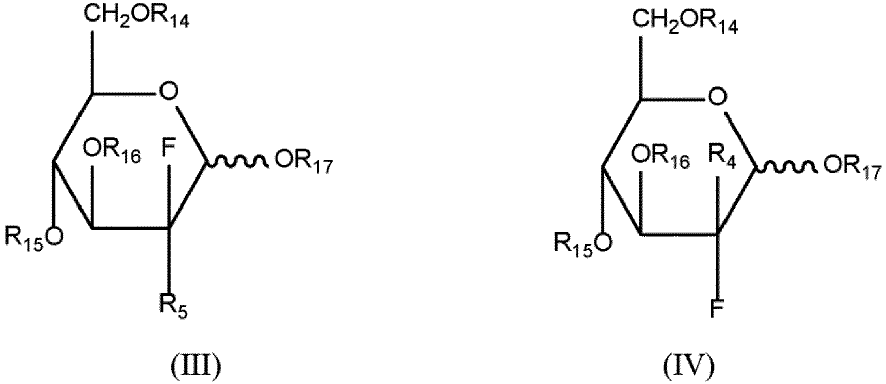 wherein:
   R₁₄, R₁₅, R₁₆, and R₁₇ are independently selected from the group consisting of hydrogen, COCH₃, COCH₂CH₃, and COCH₂CH₂CH₃ (in particular wherein R₁₄, R₁₅, R₁₆, and R₁₇ are hydrogen),
   R₄ and R₅ are independently selected from the group consisting of hydrogen, hydroxyl, thiol, halogen, alkoxy, haloalkoxy, per haloalkoxy, alkoxyalkyloxy, -OC(O)alkyl, OCO₂alkyl, alkylthio, amino, alkylamino, N-sulfonamido, N-amido, azido, carbamate, alkyl, haloalkyl, perhaloalkyl, -N(R₇)OR₈, -ON(R₉)₂, - N(R₁₀)N(R₁₁)₂, any of which may be optionally substituted, or R₄ and R₅, taken together, are selected from the group consisting of =N-OR₁₂ and =N-N(R₁₃)₂, and
   R₇, R₈, R₉, R₁₀, R₁₁, R₁₂, and R₁₃ are independently selected from the group consisting of hydrogen and alkyl, wherein said alkyl may be optionally substituted.
Embodiment 20. The pharmaceutical dispenser of embodiment 19, wherein at least one, preferably at least two, more preferably at least three of R₁₄, R₁₅, R₁₆, and R₁₇ are hydrogen.
Embodiment 21. The pharmaceutical dispenser of embodiment 20, wherein R₁₄, R₁₅, R₁₆, and R₁₇ are hydrogen.
Embodiment 22. The pharmaceutical dispenser of any one of embodiments 19 to 21, R₄ and R₅ are independently selected from the group consisting of hydrogen, hydroxyl, thiol, halogen, alkoxy, haloalkoxy, per haloalkoxy, alkoxyalkyloxy, -OC(O)alkyl, OCO₂alkyl, alkylthio, amino, alkylamino, N-sulfonamido, N-amido, azido, carbamate, alkyl, haloalkyl and perhaloalkyl.
Embodiment 23. The pharmaceutical dispenser of embodiment 22, wherein R₄ and R₅ are halogen, preferably F.
Embodiment 24. The pharmaceutical dispenser of any one of embodiments 1 to 23, wherein the analogues have any one of structural formulas V-XIII: 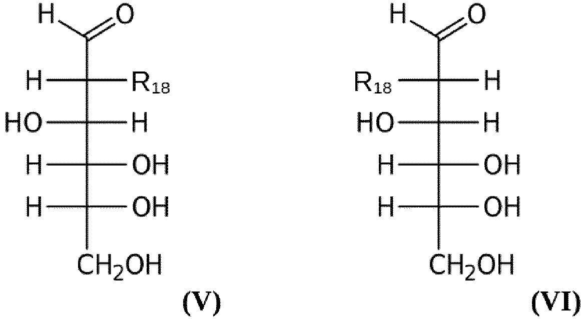 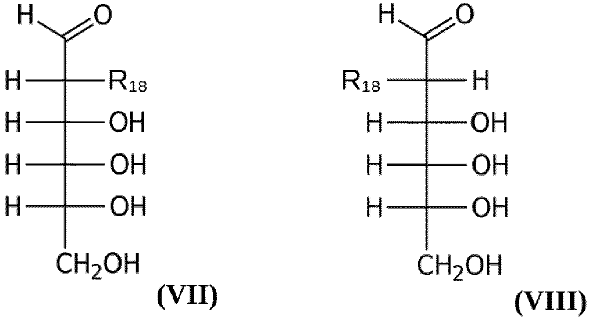 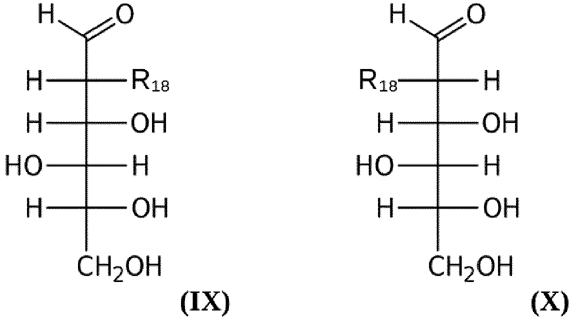 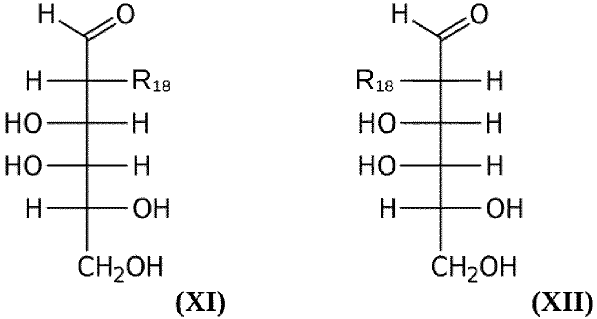 wherein R₁₈ is any one of H, F, Cl, Br, I, SH, Me, OMe, and SMe, in particular H or F.
Embodiment 25. The pharmaceutical dispenser of embodiment 24, wherein the analogues have structural formula V, wherein R₁₈ is any one of H, F, Cl, Br, I, SH, Me, OMe, azido and SMe.
Embodiment 26. The pharmaceutical dispenser of embodiment 24, wherein the analogues have structural formula VI, wherein R₁₈ is any one of H, F, Cl, Br, I, SH, Me, OMe, azido and SMe.
Embodiment 27. The pharmaceutical dispenser of any one of embodiments 1 to 26, wherein the API is an aldohexose, preferably a D-aldohexose, wherein the hydroxyl group at carbon 2 of the aldohexose is replaced by any one of H, F, Cl, Br, I, SH, Me, OM, SMe and azido.
Embodiment 28. The pharmaceutical dispenser of embodiment 27, wherein the aldohexose is a mannose or glucose, preferably D-glucose or D-mannose.
Embodiment 29. The pharmaceutical dispenser of any one of embodiments 1 to 28, wherein the API is selected from the group consisting of 2-DG, 2-chloro-2-deoxy-glucose, 2-fluoro-2-deoxy-glucose, 2-azido-2-deoxy-glucose, 2-chloro-2-deoxy-mannose, 2-fluoro-2-deoxy-mannose and 2-azido-2-deoxy-mannose, in particular selected from the group consisting of 2-DG, 2-fluoro-2-deoxy-glucose and 2-fluoro-2-deoxy-mannose; preferably wherein the glucose is D-glucose and the mannose is D-mannose.
Embodiment 30. A pharmaceutical composition for use in prevention or treatment of an infection (in particular an infection of the respiratory tract), wherein the composition is administered mucosally, wherein the pharmaceutical composition is defined according to any one of embodiments 1 to 29.
Embodiment 31. The pharmaceutical composition for use according to embodiment 30, for use in prevention or treatment of the common cold, a rhinitis or a lower respiratory tract infection, especially in an immunosuppressed individual or in an individual having COPD, cystic fibrosis or asthma.
Embodiment 32. A method for mucosally administering a pharmaceutical composition, the method comprising the steps of
   - obtaining the pharmaceutical composition, and
   - mucosally administering the composition to an individual having an infection or at risk of developing an infection;

   wherein the pharmaceutical composition comprises an API selected from the group consisting of 2-deoxy-glucose (2-DG) and analogues thereof, or a pharmaceutically acceptable salt of the API,
   wherein the pH of the pharmaceutical composition is lower than 7.4, optionally upon reconstitution in water;
   wherein the analogues have structural formula I 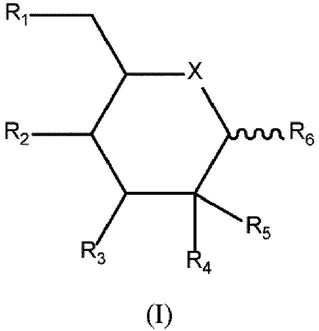
   wherein:
      X is selected from the group consisting of O and S,
      R₁, R₂, R₃, and R₆ are independently selected from the group consisting of hydrogen, hydroxyl, thiol, halogen, alkoxy, haloalkoxy, perhaloalkoxy, alkoxyalkyloxy, -OC(O)alkyl, OCO₂alkyl, alkylthio, amino, alkylamino, N-sulfonamido, N-amido, and carbamate, any of which may be optionally substituted,
      R₄ and R₅ are independently selected from the group consisting of hydrogen, hydroxyl, thiol, halogen, alkoxy, haloalkoxy, per haloalkoxy, alkoxyalkyloxy, -OC(O)alkyl, OCO₂alkyl, alkylthio, amino, alkylamino, N-sulfonamido, N-amido, azido, carbamate, alkyl, haloalkyl, perhaloalkyl, -N(R₇)OR₈, -ON(R₉)₂, - N(R₁₀)N(R₁₁)_{2'} any of which may be optionally substituted, or R₄ and R₅, taken together, are selected from the group consisting of =N-OR₁₂ and =N-N(R₁₃)₂, and
      R₇, R₈, R₉, R₁₀, R₁₁, R₁₂, and R₁₃ are independently selected from the group consisting of hydrogen and alkyl, wherein said alkyl may be optionally substituted,
      with the proviso that if X is O and R₄ and R₅ are both H, then at least one of R₁, R₂, R₃, and R₆ is not OH;
      preferably wherein the pharmaceutical composition is defined according to any one of embodiments 1 to 31.

The present invention is further illustrated by the following figures and examples, without being restricted thereto.
**Fig. 1****:** Imine formation of 2-deoxy-D-glucose (2-DG) with L-lysine in citric acid•Na₂HPO₄ buffer (pH 7) at different 2-DG concentrations incubated at 37 °C. Indicated values are means (n=4) ± SEM.
**Fig. 2****:** Imine formation of 2-deoxy-D-glucose (2-DG, 35mg/mL) with L-lysine (O.lmg/mL) in citric acid•Na₂HPO₄ buffer at different pH values incubated at 37 °C. Indicated values are means (n=4) ± SEM.
**Fig. 3****:** Imine formation of 2-deoxy-D-glucose (2-DG, 35mg/mL) with L-lysine (O.lmg/mL) in citrate buffer at different pH values incubated at 37 °C. Indicated values are means (n=4) ± SEM.
**Fig. 4****:** Imine formation of 2-deoxy-D-glucose (2-DG, 35mg/mL) with L-lysine (O.lmg/mL) in tartrate buffer at different pH values incubated at 37 °C. Indicated values are means (n=4) ± SEM.
**Fig. 5****:** Imine formation of 2-deoxy-D-glucose (2-DG, 35mg/mL) with L-lysine (O.lmg/mL) in maleate buffer at different pH values incubated at 37 °C. Indicated values are means (n=4) ± SEM.
**Fig. 6****:** Mucus penetration of 2-deoxy-D-glucose (2-DG), 2-fluoro-2-deoxy-D-glucose (2-FDG) or 2-fluoro-2-deoxy-D-mannose (2-FDM) (all 35mg/mL) in citrate buffer at pH5 and pH7, incubated for 30min at 37 °C.

### Examples

### Example 1 - Observations regarding imine formation and mucus penetration

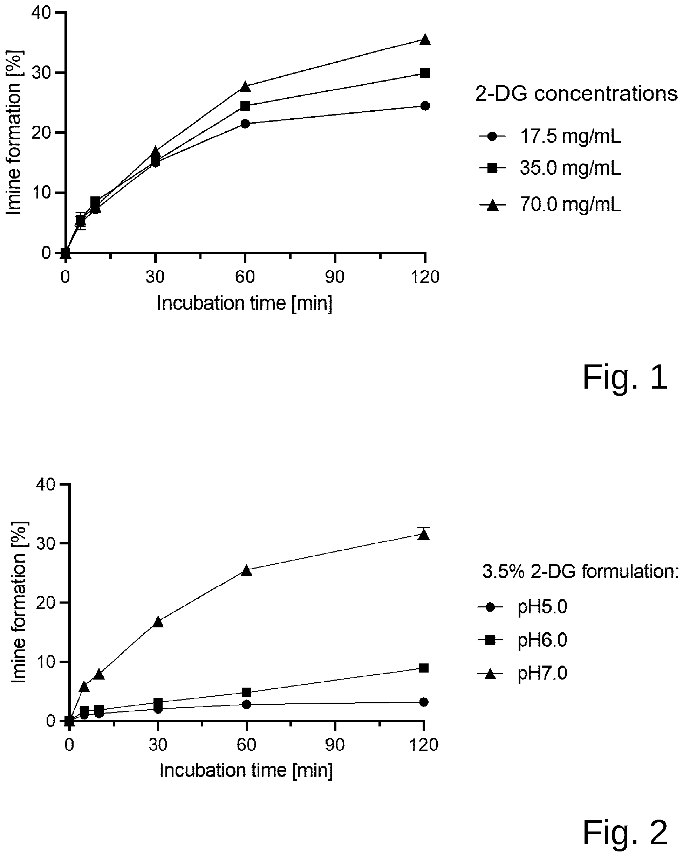

With many modes of mucosal administration, the mucosa is exposed only briefly to the drug product (in this case, 2-DG and analogues thereof). We observed that the interaction of 2-DG with free amines in the nasal mucus (modelled by the presence of L-lysine) lead to the formation of Schiff's bases (imines) (Fig. 1). This results in reduced bioavailability of 2-DG at epithelial cells, the site of infections to be targeted by 2-DG.

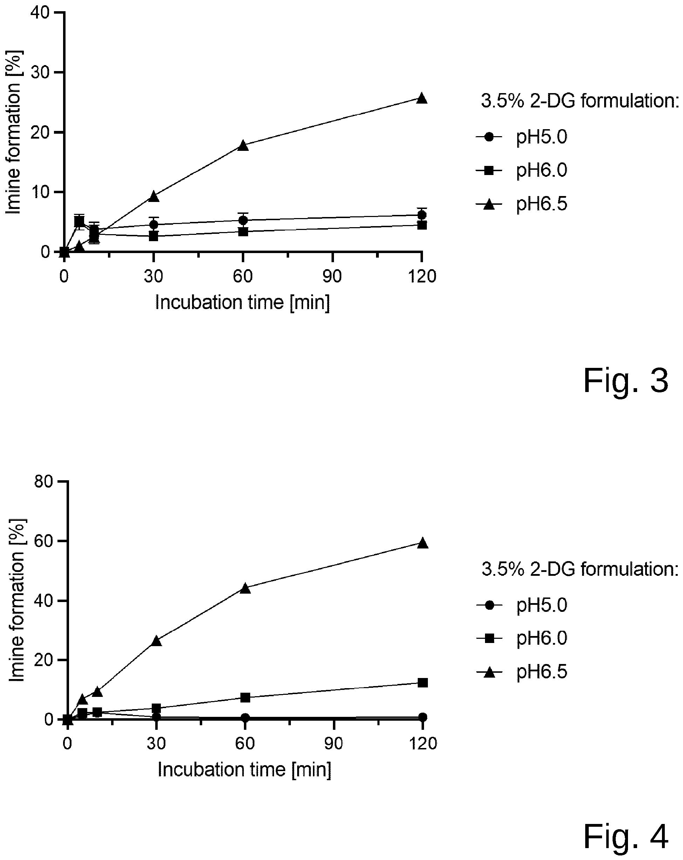

Our data showed that acidic pH buffer formulations (pH < 7.0) lead to a reduced interaction of 2-DG with free amines of the nasal mucus. This was demonstrated by the distinct lowering in the formation of Schiff's bases when testing different buffer systems with pH < 7.0 (Fig. 2-5).

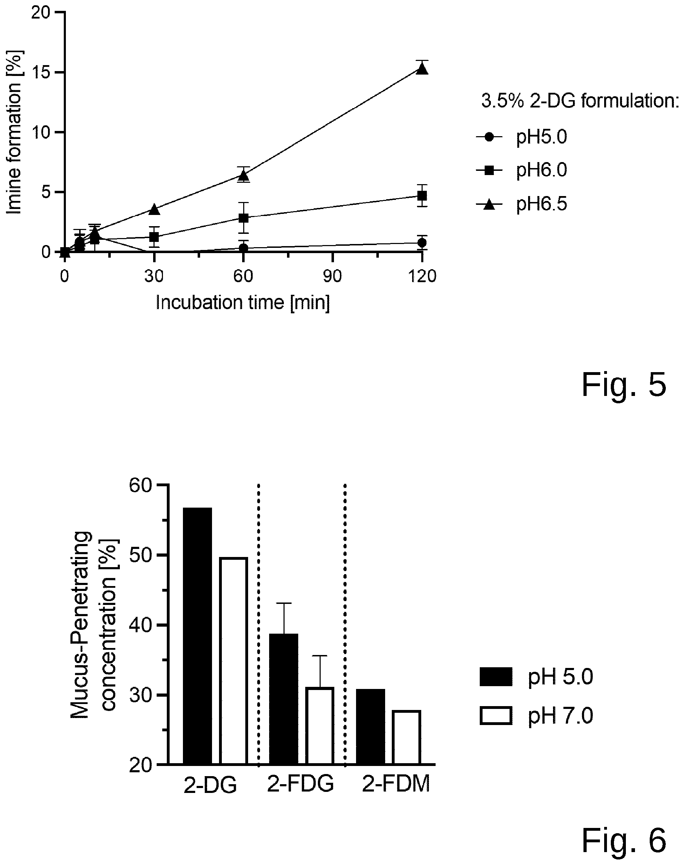

We measured the mucus penetration potential in the following manner: After applying mucus onto a semipermeable membrane, formulations containing 2-DG or analogues at indicated pH were applied onto the mucus. Penetrated 2-DG or analogues was measured via mass spectrometry. We observed that acidic pH in all tested buffer system showed increased mucus penetration properties allowing for a higher bioavailability of 2-DG, 2-fluoro-2-deoxy-D-glucose (2-FDG) and 2-fluoro-2-deoxy-D-mannose (2-FDM). See Fig. 6.

### Example 2

A metered-dose inhaler is provided containing the following pharmaceutical composition: 200 mM 2-DG, 8 mM citrate as buffering agent, 0.1% w/w benzalkonium chloride and 0.2% w/v propylene glycol as preservatives, in sterile deionised water, pH 6.0.

### Example 3

An aqueous nebulizer solution is formulated with 100 mM 2-fluoro-2-deoxy-D-glucose, 20 mM phosphate buffer, and 0.15% w/w phenylcarbinol, pH is adjusted to 5.6.

### Example 4

A nose drop applicator is provided containing a pharmaceutical composition with 250 mM 2-fluoro-2-deoxy-D-glucose, 30 mM acetate buffer, and 0.2% w/v polysorbate 80, in sterile deionised water, pH 6.4.

### Example 5

A nasal spray is provided with an aqueous pharmaceutical composition: 150 mM 2-deoxy-D-glucose, 12.5 mM citrate buffer, and 0.15% w/v polyethylene glycol, pH 5.0.

### Example 6

A metered-dose inhaler is provided, containing the following pharmaceutical composition: 700 mM 2-fluoro-2-deoxy-D-mannose, 25 mM Tris/acetate buffer, and 0.1% w/v Tween 80, in sterile deionised water, pH 5.4.

### Example 7

An aqueous nebulizer solution is formulated with 400 mM compound of example 30 of WO 2021/188586 A1 (4-fluoro-D-glucose), 10 mM maleate buffer, pH 6.2.

### Example 8

A nose drop applicator is provided with the following pharmaceutical composition: 350 mM 2-chloro-2-deoxy-glucose, 35 mM boric acid/borate buffer, and 0.15% w/v citrus oil, in sterile deionised water, pH 6.8, also containing a decongestant as an additional active agent.

### Example 9

A nasal spray is provided containing 450 mM 2-azido-2-deoxy-glucose, 50 mM ascorbate buffer in water, pH 6.0.

### Example 11

A metered-dose inhaler is provided which contains the following pharmaceutical composition: 550 mM 2-chloro-2-deoxy-glucose, 45 mM succinate buffer, in sterile deionised water, pH 5.8.

### Example 12

A nebulizer solution is formulated with 300 mM compound of example 65 of WO 2021/188586 A1, 20 mM phthalate buffer, and 0.2% w/v propylene glycol, in an aqueous solution, pH 5.8.

### Example 15

A nasal spray is provided containing the following pharmaceutical composition: 650 mM 2-DG, 30 mM citrate buffer, and 0.15% w/v polysorbate 80, in sterile deionised water, pH 5.6. The pharmaceutical composition also contains the decongestant xylometazoline as an additional active agent (0.1% w/w) .

### Example 16

A nasal spray as e.g. disclosed in US patent no. 6,000,580 is provided containing 500 mM 2-DG, 40 mM phosphate buffer, 0.9% w/v NaCl, and 0.1% w/v benzalkonium chloride, in water, pH 6.2.

### Example 17

A metered-dose inhaler containing the following pharmaceutical composition is provided: 900 mM 2-DG, 35 mM acetate buffer, and 0.05% w/v phenylcarbinol, in sterile deionised water, pH 6.0.

### Example 18

An aqueous nebulizer solution is formulated with 750 mM 2-DG, 15 mM Tris buffer, and 0.2% w/v chlorobutanol, pH 5.9.

### Example 20

A nasal spray is provided with the following pharmaceutical composition: 1000 mM 2-DG, 45 mM maleate buffer, and 0.15% w/v citrus oil, in sterile deionised water, pH 6.3, also containing 0.05% (w/w) oxymetazolin hydrochloride as additional active agent.

### Example 21

A nasal spray is provided containing 10 ml of the following pharmaceutical composition: 550 mM 2-deoxy-D-glucose, 0.9% w/v NaCl, pH 4.3 (acidified with HCl) in sterile deionised water.

### Example 22

A nose drop applicator as e.g., disclosed in EP 0 170 198 A2 is provided containing 5 ml of the following pharmaceutical composition: 440 mM 2-deoxy-2-fluoro-D-mannose, 0.05% (w/w) oxymetazoline hydrochloride, 0.05% (w/w) benzalkonium chloride, 40% (v/v) glycerol in 100 mM acetate buffer (pH 5.3).

### Example 23

A nebuliser as e.g., disclosed in US 9,364,618 B2 is provided containing the following pharmaceutical composition in its fluid reservoir: 350 mM 2-deoxy-D-glucose, 0.9% w/v NaCl dissolved in 50 mM Tris/glycine buffer (pH 5.2). The nebuliser is used to deliver the composition by inhalation as an aerosol to the lower respiratory tract of an immune-suppressed patient suffering from viral pneumonia.

### Example 24

A metered-dose inhaler containing the following pharmaceutical composition is provided: 500 mM API, 35 mM citrate buffer, and 0.05% w/v phenylcarbinol, in sterile deionised water, pH 6.0. The API is selected from any one of the 2-DG analogues given in examples 1-84 of paragraph [0151] of WO 2021/188586 A1.

### Example 25

A nasal spray is provided with the following pharmaceutical composition: 700 mM API, 45 mM citrate buffer, and 0.15% w/v citrus oil, in sterile deionised water, pH 5.5, also containing 0.05% (w/w) oxymetazolin hydrochloride (decongestant) as additional active agent. The API is selected from any one of the 2-DG analogues given in examples 1-84 of paragraph [0151] of WO 2021/188586 A1.

### Example 26

An aqueous nebulizer solution is formulated with 400 mM API, 30 mM tartrate buffer, pH 5.2. The API is selected from any one of the 2-DG analogues given in examples 1-84 of paragraph [0151] of WO 2021/188586 A1.

## Claims

1. A pharmaceutical dispenser for mucosal administration, preferably intranasal or inhalatory administration, containing a pharmaceutical composition,
wherein the pharmaceutical composition comprises an active pharmaceutical ingredient (API) selected from the group consisting of 2-deoxy-glucose (2-DG) and analogues thereof, or a pharmaceutically acceptable salt of the API,
wherein the pH of the pharmaceutical composition is lower than 7.4, optionally upon reconstitution in water;
wherein the analogues have structural formula I 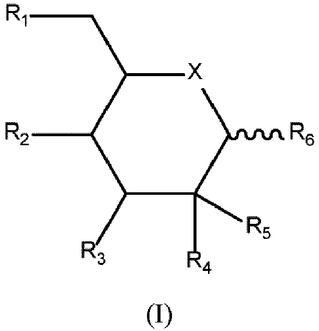
wherein:
X is selected from the group consisting of O and S, R₁, R₂, R₃, and R₆ are independently selected from the group consisting of hydrogen, hydroxyl, thiol, halogen, alkoxy, haloalkoxy, perhaloalkoxy, alkoxyalkyloxy, -OC(O)alkyl, OCO₂alkyl, alkylthio, amino, alkylamino, N-sulfonamido, N-amido, and carbamate, any of which may be optionally substituted,
R₄ and R₅ are independently selected from the group consisting of hydrogen, hydroxyl, thiol, halogen, alkoxy, haloalkoxy, per haloalkoxy, alkoxyalkyloxy, -OC(O)alkyl, OCO₂alkyl, alkylthio, amino, alkylamino, N-sulfonamido, N-amido, azido, carbamate, alkyl, haloalkyl, perhaloalkyl, -N(R₇)OR₈, -ON(R₉)₂, - N(R₁₀)N(R₁₁)_{2'} any of which may be optionally substituted, or R₄ and R₅, taken together, are selected from the group consisting of =N-OR₁₂ and =N-N(R₁₃)₂, and
R₇, R₈, R₉, R₁₀, R₁₁, R₁₂, and R₁₃ are independently selected from the group consisting of hydrogen and alkyl, wherein said alkyl may be optionally substituted,
with the proviso that if X is O and R₄ and R₅ are both H, then at least one of R₁, R₂, R₃, and R₆ is not OH.

2. The pharmaceutical dispenser of claim 1, wherein the pharmaceutical dispenser is selected from the group consisting of dispensers for intranasal administration, such as nasal sprays and nose drop applicators, and inhalation devices, such as dry-powder inhalers, metered-dose inhalers and nebulizers.

3. The pharmaceutical dispenser of claim 2, wherein the pharmaceutical dispenser is a nasal spray and the pharmaceutical composition is an aqueous solution.

4. The pharmaceutical dispenser of any one of claims 1 to 3, wherein the pH of the pharmaceutical composition is 3.0 to 7.0, preferably 3.2 to 6.8 or even 3.4 to 6.6, more preferably 3.6 to 6.4 or even 3.8 to 6.2, even more preferably 4.0 to 6.0.

5. The pharmaceutical dispenser of any one of claims 1 to 4, wherein the pharmaceutical composition comprises a buffering agent.

6. The pharmaceutical dispenser of claim 5, wherein the buffering agent comprises a weak acid with a pKₐ of 3.0 to 7.0, preferably 3.5 to 6.5, more preferably 4.0 to 6.0, especially 4.5 to 5.5.

7. The pharmaceutical dispenser of claim 5 or 6, wherein the buffering agent is selected from the group consisting of citrate, phosphate, acetate, citrate/phosphate, tris(hydroxymethyl)aminomethane (Tris), Tris/glycine, Tris/acetate, maleate, tartrate, boric acid/borate, ascorbate, succinate and phthalate, in particular citrate, citrate/phosphate and tartrate.

8. The pharmaceutical dispenser of any one of claims 1 to 7, wherein the analogues have any one of structural formulas V-XIII: 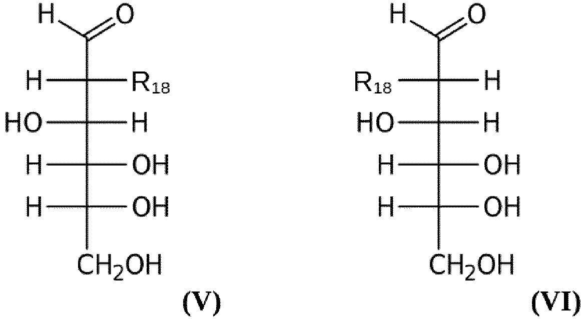 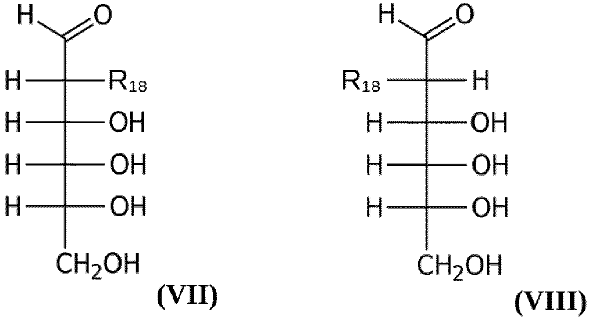 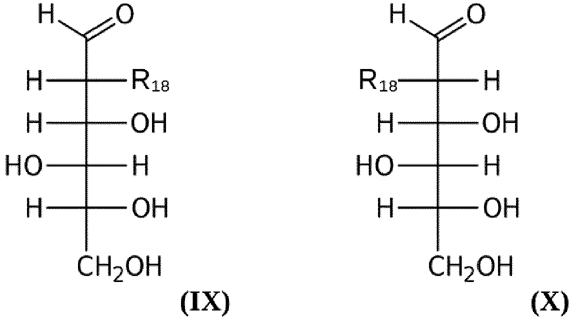 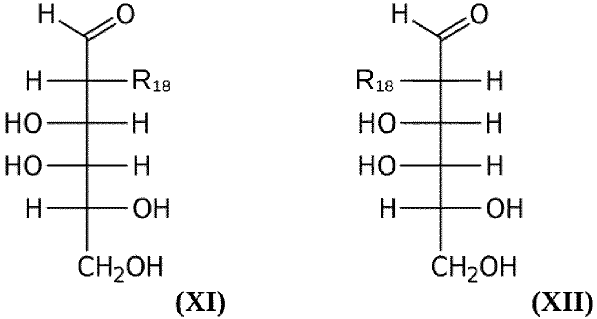 wherein R₁₈ is any one of H, F, Cl, Br, I, SH, Me, OMe, and SMe, in particular H or F.

9. The pharmaceutical dispenser of claim 8, wherein the analogues have structural formula V, wherein R₁₈ is any one of H, F, Cl, Br, I, SH, Me, OMe, azido and SMe.

10. The pharmaceutical dispenser of claim 8, wherein the analogues have structural formula VI, wherein R₁₈ is any one of H, F, Cl, Br, I, SH, Me, OMe, azido and SMe.

11. The pharmaceutical dispenser of any one of claims 1 to 10, wherein the API is selected from the group consisting of 2-DG, 2-chloro-2-deoxy-glucose, 2-fluoro-2-deoxy-glucose, 2-azido-2-deoxy-glucose, 2-chloro-2-deoxy-mannose, 2-fluoro-2-deoxy-mannose and 2-azido-2-deoxy-mannose, in particular selected from the group consisting of 2-DG, 2-fluoro-2-deoxy-glucose and 2-fluoro-2-deoxy-mannose; preferably wherein the glucose is D-glucose and the mannose is D-mannose.

12. The pharmaceutical dispenser of any one of claims 1 to 11, wherein the API is 2-DG.

13. A pharmaceutical composition for use in prevention or treatment of an infection, wherein the composition is administered mucosally, wherein the pharmaceutical composition is defined according to any one of claims 1 to 12.

14. The pharmaceutical composition for use according to claim 13, for use in prevention or treatment of the common cold, a rhinitis or a lower respiratory tract infection, especially in an immunosuppressed individual or in an individual having COPD, cystic fibrosis or asthma.

15. A method for mucosally administering a pharmaceutical composition, the method comprising the steps of
- obtaining the pharmaceutical composition, and
- mucosally administering the composition to an individual having an infection or at risk of developing an infection;
wherein the pharmaceutical composition comprises an API selected from the group consisting of 2-DG and analogues thereof, or a pharmaceutically acceptable salt of the API,
wherein the pH of the pharmaceutical composition is lower than 7.4, optionally upon reconstitution in water;
wherein the analogues have structural formula I 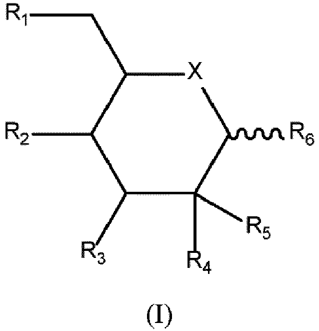
wherein:
X is selected from the group consisting of O and S, R₁, R₂, R₃, and R₆ are independently selected from the group consisting of hydrogen, hydroxyl, thiol, halogen, alkoxy, haloalkoxy, perhaloalkoxy, alkoxyalkyloxy, -OC(O)alkyl, OCO₂alkyl, alkylthio, amino, alkylamino, N-sulfonamido, N-amido, and carbamate, any of which may be optionally substituted,
R₄ and R₅ are independently selected from the group consisting of hydrogen, hydroxyl, thiol, halogen, alkoxy, haloalkoxy, per haloalkoxy, alkoxyalkyloxy, -OC(O)alkyl, OCO₂alkyl, alkylthio, amino, alkylamino, N-sulfonamido, N-amido, azido, carbamate, alkyl, haloalkyl, perhaloalkyl, -N(R₇)OR₈, -ON(R₉)₂, - N(R₁₀)N(R₁₁)_{2'} any of which may be optionally substituted, or R₄ and R₅, taken together, are selected from the group consisting of =N-OR₁₂ and =N-N(R₁₃)₂, and
R₇, R₈, R₉, R₁₀, R₁₁, R₁₂, and R₁₃ are independently selected from the group consisting of hydrogen and alkyl, wherein said alkyl may be optionally substituted,
with the proviso that if X is O and R₄ and R₅ are both H, then at least one of R₁, R₂, R₃, and R₆ is not OH.
